# EUROPEAN PATENT SPECIFICATION

(11) **EP 3 607 981 B1**
(45) Date of publication and mention of the grant of the patent: **23.10.2024**
(21) Application number: 18781032.0
(22) Date of filing: 03.04.2018
(51) Int. Cl.: A61M 5/19, A61J 1/20, A61M 5/28, A61M 5/24, A61M 1/00, A61M 35/00, A61M 5/14, A61L 26/00, A61F 13/00, A61K 9/00, A61K 47/02, A61K 47/34, A61K 47/36, A61P 23/02

(54) **KIT FOR TREATING OR RELIEVING PAIN AT INCISION SITE FOLLOWING SURGICAL PROCEDURE**
KIT ZUR BEHANDLUNG ODER LINDERUNG VON SCHMERZEN AN EINER INZISIONSSTELLE NACH EINEM CHIRURGISCHEN EINGRIFF
KIT POUR LE TRAITEMENT OU LE SOULAGEMENT DE LA DOULEUR AU NIVEAU D'UN SITE D'INCISION APRÈS UNE INTERVENTION CHIRURGICALE

(30) Priority: 04.04.2017 KR 20170043850
(43) Date of publication of application: 12.02.2020
(73) Proprietor: Genewel Co., Ltd, Seongnam-si, Gyeonggi-do 13211 (KR)
(72) Inventor: PARK, Il Kyu, Seongnam-si Gyeonggi-do 13203 (KR); KIM, Jun Ho, Gwangju-si, Gyeonggi-do 12736 (KR); CHOI, Jong Bae, Seongnam-si, Gyeonggi-do 13588 (KR); KIM, Hun Ui, Suwon-si, Gyeonggi-do 16435 (KR)
(74) Representative: Dehns
(86) International application number: PCT/KR2018/003921
(87) International publication number: WO 2018/186658

(56) References cited:
- EP-A1- 0 470 703
- WO-A1-2009/073658
- WO-A1-2018/186658
- KR-A- 20070 100 701
- KR-B1- 100 565 881
- KR-B1- 101 125 934
- KR-B1- 101 503 663
- US-A- 5 939 485

## Description

### [Technical Field]

The present invention relates to a kit for relieving or treating postoperative incision site pain, and more particularly to a kit for relieving or treating incision site pain, which makes effective treatment possible by injecting a pain relief drug or a treatment drug into a surgical site *in situ* after incisional surgery during an incisional surgical procedure, and stably and slowly releasing the drug.

### [Background Art]

During a surgical procedure, an incision site is injected with a pain relief drug such as a local anesthetic for the purpose of relieving postoperative pain and is injected with drugs such as antibiotics and anti-inflammatory drugs for therapeutic purposes.

However, since most pain-relieving drugs are non-viscous solutions and a washing solution is usually used during incisional surgery, it is difficult to quickly and effectively produce the intended drug effect quickly and effectively by accurately and stably injecting the pain relief drug into a target incision site. In addition, the antibiotics and anti-inflammatory drugs are not effective due to their short half-life.

Accordingly, various wound dressings based on body temperature-responsive polymers have been developed, and the applicant of the present invention also has filed a patent application related to a drug-containing wound dressing (Korean Patent No. 10-1125934).

WO 2009/073658 A1 discloses a composition for the treatment of surgical incisions comprising: a drug, 15-20 wt% of poloxamer 407, the composition has a viscosity at room temperature of from 100,000 to 1,000,000 mPa.s. The composition is applied through a syringe with a needle.

However, various pain relief drugs or treatment drugs may be required depending on the size or condition of an incision site or on the progress of surgery, and if necessary, a mixture of two or more thereof needs to be used. However, a wound dressing obtained by mixing all kinds of drugs together at various mixing ratios in view of this fact cannot be prepared in advance, and even if this wound dressing is prepared in advance, purchasing each of various kinds of drugs is economically undesirable in terms of purchase costs, storage costs, etc.

### [Disclosure]

### [Technical Problem]

Therefore, it is an object of the present invention to provide a kit for treating or relieving postoperative incision site pain, which may overcome conventional problems occurring when injecting drugs for treating or relieving surgical incision site pain and may quickly and effectively produce the intended drug effect by accurately and stably injecting a drug into a target incision site.

The above and other objects of the present invention can all be achieved by the present invention described below.

### [Technical Solution]

To achieve the above object, the present invention provides a surgical kit as defined in claim 1. a first syringe 200 configured to be filled with the pain relief or treatment drug immediately before use so as to prepare the mixture solution; and a syringe connector 400 configured to mix the substances filled in the first syringe and the prefilled syringe, respectively.

In one embodiment, the surgical kit may further include a syringe needle 201 for the first syringe.

In one embodiment, the surgical kit may further include a syringe needle 202 for the prefilled syringe.

In one embodiment, the temperature-responsive viscous solution may be a non-pyrogenic viscous solution including the polyethylene-polypropylene-polyethylene polymer, alginic acid sodium alginate, calcium chloride, and water for injection.

In the present description, the term "non-pyrogenic viscous solution" means that there is no heat generation during temperature-dependent so-gel phase transition. As a specific example, the term means that the temperature change during the phase transition is 5°C or less, 3°C or less, or 1°C or less.

In one embodiment, the first syringe may be preassembled with a syringe needle or connected with the syringe needle immediately before use, and the syringe needle may be separated from the first syringe after being filled with the pain relief drug.

In one embodiment, the mixture solution injection guide tube may be connected to the prefilled syringe such that the mixture solution in the prefilled syringe may be injected into the incision site.

In one embodiment, the syringe connector may have an inner diameter of 10 mm or less, through which the drug passes.

In one embodiment, the surgical kit may further include an absorption means for absorbing and removing water remaining around the incision site.

The surgical kit according to the present invention can be used in a method including the steps of: connecting a syringe needle to a first syringe, inserting the syringe needle into the pain relief or treatment drug to be used during surgery, filling the drug into the first syringe up to a marked line, and then separating the connected syringe needle; removing a stopper from the prefilled syringe prefilled with the temperature-responsive viscous solution and equipped with a piston; connecting the prefilled syringe, from which the stopper was removed, to one side of a syringe connector; connecting the first syringe, which contains the drug filled therein and from which the syringe needle was separated, to the other side of the syringe connector; mixing the drug with the temperature-responsive viscous solution in the prefilled syringe by using the piston of each of the first syringe and the prefilled syringe, which communicate with each other by the syringe connector; and after the mixing, separating the prefilled syringe, filled with the mixture solution, from the syringe connector, inserting the mixture solution injection guide tube or a syringe needle into the prefilled syringe, and applying the mixture solution to the surgical incision site by injecting the mixture solution into the surgical incision site.

The method may further include, before injecting the mixture solution into the surgical incision site, a step of sucking and removing a washing solution used during the surgery by a suction means.

The surgical kit for use in treating or relieving incision site pain, may also include a first syringe 200 configured to be filled with the pain relief or treatment drug immediately before use so as to prepare the mixture solution; and a syringe connector 400 configured to mix the substances filled in the first syringe and the prefilled syringe, respectively, wherein the temperature-responsive viscous solution additionally includes 0.005 to 0.1 wt% of Cacl₂ as a crosslinking agent.

The alginate may be a metal alginate, preferably an alkali metal alginate or an alkaline earth metal alginate, most preferably an alkali metal alginate.

### [Advantageous Effects]

The present invention configured as described above may provide a kit for treating and reducing (relieving/killing) postoperative incision site pain, which makes it possible to mix a necessary pain relief drug or treatment drug with a temperature-responsive viscous solution by a simple procedure *in situ* after incisional surgery during an incisional surgical procedure, and makes effective treatment possible by injecting the drug-c containing mixture into a surgical site and stably and slowly releasing the drug.

### [Description of Drawings]

FIG. 1 is a photograph showing the overall configuration of a kit for treating or relieving incision site pain according to the present invention.
FIG. 2 is a schematic view showing an embodiment in which a surgical kit of the present invention is assembled and used, with the passage of time.
FIG. 3 is a graph showing the results of a stability test (A) for a temperature-responsive viscous solution, contained in a pain relief drug-containing mixture solution as a function of time.
FIG. 4 is a graph showing the results of a slow-release test for a pain relief drug of a pain relief drug-containing mixture solution as a function of time.
FIG. 5 shows the pain-reducing/pain-relieving effects of a surgical kit on rat paw pain-induced models, obtained when a pain relief drug and a temperature-responsive viscous solution were not used, when the pain relief drug was used alone, when the temperature-responsive viscous solution was used alone, and when a pain relief drug-containing mixture solution was used, as a function of time.
FIG. 6 shows the results of a stability test performed depending on whether the temperature-responsive viscous solution (containing 30 wt% of a temperature-responsive polymer) contained in a pain relief drug-containing mixture solution was crosslinked, as a function of time.
FIG. 7 shows the results for a precipitation test for a pain relief drug-containing mixture solution (left) and a treatment drug-containing mixture solution (right), as a function of time.
FIG. 8 shows the results of a slow-release test for a pain relief drug (ibuprofen) of a pain relief drug-containing mixture solution, as a function of time.
FIGS. 9 and 10 show the results of a slow-release test performed while changing the kinds of drug (ropivacaine vs. bupivacaine) and viscous solution (crosslinked vs. non-crosslinked) in a pain relief drug-containing mixture solution, as a function of time.

### [Mode for Invention]

The present invention provides a surgical kit for treating or relieving incision site pain as defined in claim 1.

The kit for relieving incision site pain may also comprise: a first syringe configured to be filled with a pain relief drug immediately before use so as to prepare the pain relief drug-containing mixture solution; and a syringe connector configured to mix the substances filled in the first syringe and the prefilled syringe, respectively, the syringe connector having an opening/closing means.

Hereinafter, preferred embodiments of a kit 10 for alleviating incision site pain according to the present invention will be described in detail with reference to the accompanying drawings.

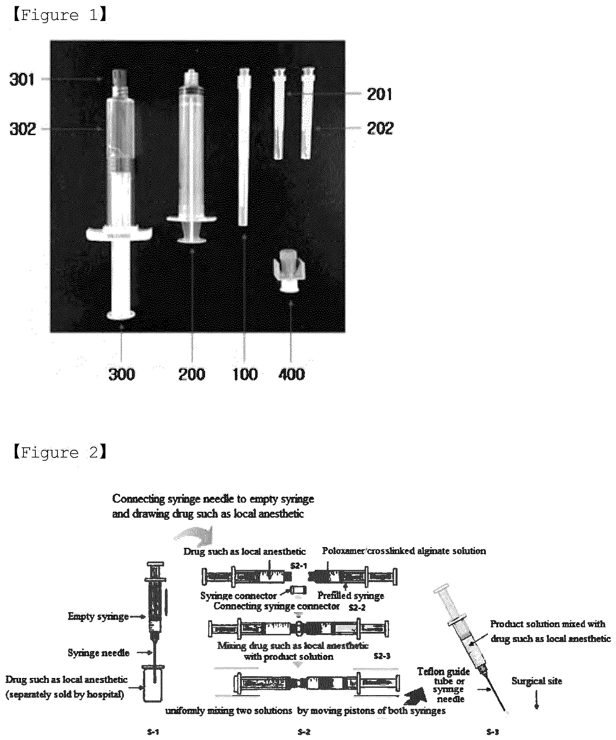

FIG. 1 illustrates a kit 10 for relieving incision site pain according to the present invention (hereinafter referred to as the surgical kit of the present invention").

As illustrated in FIG. 1, the surgical kit 10 of the present invention includes a mixture solution injection guide tube 100, a first syringe 200, a prefilled syringe 300, a syringe connector 400, and syringe needles 201 and 202.

Here, all the components included in the surgical kit 10 of the present invention may access incision sites during various surgical procedures, and thus are configured such that they may be applied to incision sites *in situ* after surgical operations.

The mixture solution injection guide tube 100 serves to inject a pain relief drug-containing mixture solution in close proximity to an exposed incision site during a surgical operation. It may be composed of a both-end-open type tube made of a flexible material such as Teflon. For example, it may be composed of a Teflon capillary tube.

The mixture solution injection guide tube 100 may be configured such that one end thereof may be inserted into the inlet of the prefilled syringe and the mixture solution may be discharged or injected through the other end.

The mixture solution injection guide tube 100 has, for example, a total length of 60 to 70 mm, preferably 70 ± 3.5 mm, an outer diameter of 1.6 to 1.8 mm, preferably 1.7 ± 0.085 mm, and an inner diameter of 1.1 to 1.4 mm, preferably 1.26 ± 0.085 mm. Within these ranges, the mixture solution injection guide tube is easy to handle and convenient to use, and the effect of appropriately distributing the drug in the surgical site is great.

The first syringe 200 is configured to be filled with a pain relief drug (not shown) immediately before use so as to prepare the pain relief drug-containing mixture solution. The first syringe 200 may be preassembled with a syringe needle 201 before use, or may be connected with the syringe needle 201 immediately before use. After the first syringe 200 is filled with a pain relief drug (not shown), the syringe needle 201 is separated therefrom. As the first syringe 200, any commercially available product equipped with a piston is preferably used.

As the pain relief drug, a local anesthetic, an opiate analgesic, a nonsteroidal drug are used for the purpose of controlling postoperative acute pain. For example, ropivacaine hydrochloride, ibuprofen or the like may be used which is relatively safe.

The pain relief drug selected from a local anesthetic, an opiate analgesic, a nonsteroidal drug, is not particularly limited as long as it dissolves in water, is stable in an aqueous solution, and may be used as an injection. For example, it may be ibuprofen.

In the present description, when the drug is intended for both pain relief and treatment, it may be classified as either a pain relief drug or a treatment drug.

The prefilled syringe 300 preferably has a structure which is opened and closed by a stopper 301, instead of a syringe needle which is generally connected.

As a specific example, the prefilled syringe 300 is filled with a temperature-responsive viscous solution 302 acting as a matrix for the pain relief drug-containing mixture solution, has a structure which is opened and closed by a stopper, and is stable to autoclaving.

Here, the reason why the temperature-responsive viscous solution is pre-filled is to prevent the user's mistakes from occurring during the filling process, provide convenience during use in an operating room, and shorten the product production time.

In this regard, the temperature-responsive viscous solution 302 is a matrix which is changed to a gel state by the body temperature after application to a surgical incision site and plays an important role in providing stability and sustained-release properties. The temperature-responsive viscous solution is composed of an ionically crosslinked alginate and a temperature-responsive poly(ethylene oxide)/poly(propylene oxide)/poly(ethylene oxide) triblock copolymer. It is preferably composed of a mixture further comprising a trace amount of CaCl₂, and water for injection.

In the present description, the term "ionically crosslinked alginate" may refer to an alginic acid or alginate crosslinked by, for example, a crosslinking agent such as CaCl₂.

In the present description, viscosity (cps) is measured with a Brookfield viscometer under conditions of #4 spindle at 5°C and #7 spindle at 37°C in accordance with method 2 (rotational viscometer method) described in the Korean Pharmacopoeia.

At 5°C, the temperature-responsive viscous solution 302 has a viscosity of 50 to 5,000 cps (1 cps corresponds to 1 mPA·s), 100 to 5,000 cps, or 500 to 3,000 cps, preferably 500 to 1,000 cps, and is easily mixed with a pain relief agent such as a local anesthetic for controlling postoperative acute pain. The temperature-responsive viscous solution is gelled *in vivo,* and allows the pain relief drug to be stably and slowly released to a target site.

As another example, at 5°C, the temperature-responsive viscous solution 302 has a viscosity of 50 to 3,000 cps, or 100 to 2,000 cps, preferably 100 to 1,000 cps, and is easily mixed with a pain relief agent such as a local anesthetic for controlling postoperative acute pain.

The temperature-responsive viscous solution contains an ionically crosslinked alginate in an amount of 0.05 to 3 wt%, or 0.1 to 3 wt%, preferably 0.1 to 2 wt%, based on 100 wt% of the solution. Within this range, the effect of improving the stability of the temperature-responsive viscous solution may be provided.

A crosslinking agent for the ionically crosslinked alginate may be, for example, one or more selected from among a halide having one or more cations selected from among Li⁺, Na⁺, K⁺, Rb⁺, Cs⁺, Fr⁺, Be²⁺, Ra²⁺, B³⁻, Al³⁺, Ga²⁺, Mg²⁺, Ca²⁺, Sr²⁺ and Ba²⁺, preferably one or more cations selected from among Mg²⁺, Ca²⁺, Sr²⁺ and Ba²⁺, or chitosan, glutaraldehyde, formalin, and poly-L-lysine, but is not limited.

The temperature-responsive viscous solution 302 contains the temperature-responsive poly (ethylene oxide)/poly(propylene oxide)/poly(ethylene oxide) triblock copolymer in an amount of 30 to 40 wt%. Within this range, the effect of stably maintaining the pain relief drug *in vivo* may be provided.

The poly(ethylene oxide)/poly(propylene oxide)/poly(ethylene oxide) triblock copolymer includes a poly(ethylene oxide) block and a poly(propylene oxide) block at a ratio of 90 : 105 to 50 : 70. Within this range, the effect of stably maintaining the pain relief drug *in vivo* may be provided.

Other properties of the poly(ethylene oxide)/poly(propylene oxide)/poly(ethylene oxide) triblock copolymer, such as weight-average molecular weight, are not particularly limited as long as they correspond to the properties of poly(ethylene oxide)/poly(propylene oxide)/poly(ethylene oxide) triblock copolymers which may generally be used in the technical field related to temperature-responsive viscous solutions.

The temperature-responsive viscous solution 302 may contain CaCl₂ in an amount of 0.005 to 0.1 wt% or 0.007 to 0.1 wt%, preferably 0.01 to 0.1 wt%, based on 100 wt% of the solution. Within this range, the effect of uniformly mixing the crosslinked alginate with the poly(ethylene oxide)/poly(propylene oxide)/poly(ethylene oxide) triblock copolymer may be provided.

As another example, the temperature-responsive viscous solution 302 may contain CaCl₂ in an amount of 0.005 to 0.3 wt% or 0.01 to 0.3 wt%, preferably 0.01 to 0.20 wt%, based on 100 wt% of the solution. Within this range, the effect of uniformly mixing the crosslinked alginate with the poly(ethylene oxide)/poly(propylene oxide)/poly(ethylene oxide) triblock copolymer may be provided.

The temperature-responsive viscous solution 302 has a stickiness of 0.8 N or higher, or 0.8 N to 5 N. Within this range, the effect of stably maintaining the pain relief drug *in vivo* may be provided. The stickiness (N) is measured using a rotational rheometer at 5°C.

The temperature-responsive viscous solution 302 is biocompatible to avoid problems such as a slow recovery rate of a surgical incision site or a decrease in the adhesive strength of a suture suturing the incision site, and preferably has the property of allowing the surgical incision site to be normally healed.

The syringe connector 400 serves to discharge and mix the respective substances filled in the first and prefilled syringes, and preferably has an inner diameter of 12 mm or less, 10 mm or less, 1 to 10 mm, or 1.9 to 4.1 mm, so as to enable the viscous solution to smoothly move therein. The mixing ratio between the pain relief substance (drug) or the treatment substance (drug) and the temperature-responsive viscous solution, which are mixed and introduced into the prefilled syringe through the syringe connector, may be a volume ratio of 1:0.5 to 5 (aqueous drug solution : temperature-responsive viscous solution) or 1:0.5 to 2. Within this range, the effect of stably maintaining the pain relief drug or the treatment drug *in vivo* may be provided. When the pH of the drug aqueous solution is 4 or more or 4 to 8, the volume ratio between the temperature-responsive viscous solution and the drug is 1:0.5 to 5 (aqueous drug solution : temperature-responsive viscous solution), and when the pH is pH is less than 4 or 1 to 4, the volume ratio is 1:4 to 40 (aqueous drug solution : temperature-responsive viscous solution). Within this range, the pain relief drug or the treatment drug is not released quickly *in vivo,* is stably maintained *in vivo,* and is slowly released.

In the embodiments according to the invention the concentration of a final drug mixture containing the aqueous drug solution and the temperature-responsive viscous solution is 0.1 to 0.5 wt%, or 0.2 to 0.4 wt%.

From the prefilled syringe 300 filled with the mixture through the syringe connector 400, the syringe connector 400 is separated. Then, to the part connected with the stopper 301 for the pain relief drug-containing mixture solution (not shown) in the prefilled syringe 300, the above-described mixture solution injection guide tube 100 or second syringe needle 202 is connected instead of the stopper, whereby the pain relief drug-containing mixture solution can be stably injected into a target surgical incision site. Through this accurate and stable injection of the mixture solution into the target incision site, the pain relief drug-containing mixture solution is changed to a gel state by the temperature-responsive viscous solution 302 contained therein and slowly releases the pain relief drug. For example, the gel state stably maintains its shape after about 5 minutes.

In addition, if necessary, it is also possible to absorb and remove water remaining around the incision site by using a separate absorbing means (not shown), for example, cotton swabs or gauze.

A method of using the surgical kit 10 of the present invention for a surgical incision site will now be described with reference to the accompanying drawings.

FIG. 2 is a schematic view showing an embodiment in which the surgical kit of the present invention is assembled and used, in a time-dependent manner.

Referring to FIG. 2, in step S1, in a sterilized place, the package of the surgical kit 10 is removed, and the syringe needle 201 is connected to the first syringe 200 equipped with a piston. Then, the needle 201 is inserted into the pain relief drug such as a local anesthetic to be used during surgery, and the pain relief drug is filled into the first syringe 200 up to the marked line.

Step S2 consists of a total of three steps: step S2-1, step S2-2, and step S2-3. First, in step S2-1, the syringe needle 201 connected to the first syringe 200 filled with the pain relief drug is separated (see the left side), followed by removal of the stopper 301 from the prefilled syringe 300 prefilled with the temperature-responsive viscous solution (the product DDK gel (Korea Food and Drug Administration Approval No. 09-826) marketed by the applicant of the present invention) and equipped with a piston (see the right side).

In step S2-2, the syringe connector 400 is connected to the prefilled syringe 300 from which the stopper 301 was removed, and the first syringe 200 filled with the pain relief drug is connected to the prefilled syringe. At this time, care must be taken not to allow the pain relief drug to flow down.

In step S2-3, the pain relief drug is uniformly mixed with the temperature-responsive viscous solution in the prefilled syringe 300 while the pistons of the first syringe 200 and the prefilled syringe 300 connected to each other by the syringe connector 400 are pushed from side to side.

Next, in step S3, the syringe connector 400 is separated, and then the mixture solution injection guide tube 100 or the second syringe needle 202 is connected to the prefilled syringe 300, and the pain relief drug-containing mixture solution is sufficiently injected into a surgical incision site and applied.

If necessary, the method may include, before step S1, a step of sucking and removing the washing solution used in surgery by a suction means (not shown) and confirming that sufficient hemostasis of the wound surface was made during the surgery.

In the following, only examples in which compositions obtained by mixing components as defined in claim 1 are according to the claims.

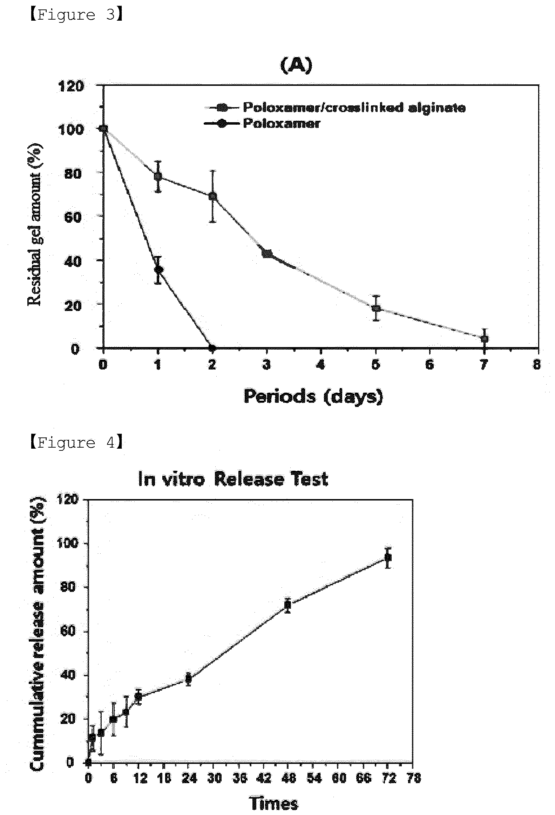

FIGS. 3 and 4 show the results of a stability test for the temperature-responsive viscous solution and a release test for the pain relief drug of the mixture of the temperature-responsive viscous solution and the pain relief drug.

The test results are summarized as follows. As shown in FIG. 3 showing the results of an *in vitro* stability test for the temperature-responsive viscous solution, it was confirmed that when the temperature-responsive viscous solution was used, the stability thereof was maintained up to 7 days, unlike a temperature-responsive polymer. As shown in FIG. 4 showing the results of a release test for the pain relief drug-containing mixture, it was confirmed that the pain relief drug was released slowly up to 3 days (72 hours).

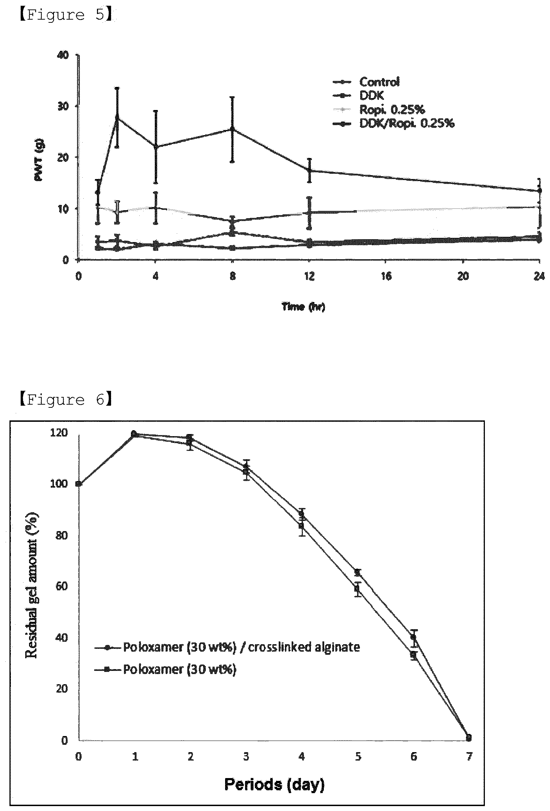

FIG. 5 shows the pain-reducing/pain-relieving effects on rat paw pain-induced models, obtained when the pain relief drug and the temperature-responsive viscous solution were not used, when the pain relief drug was used alone, when the temperature-responsive viscous solution was used alone, and when the pain relief drug-containing mixture solution was used.

In the figure, the non-use of the pain relief drug and the temperature-responsive viscous solution is marked as control; the use of the temperature-responsive viscous solution alone is marked as DDK; the use of the pain relief drug alone is marked as Ropi. (which is the abbreviation of the substance used) with concentration % (wt% concentration in aqueous solution); and the use of the pain relief drug-containing mixture solution is marked as DDK/Ropi. with concentration %.

In the present description, the concentration % means the weight % concentration unless otherwise stated.

For reference, Ropi. 0.25% means a composition containing the temperature-responsive viscous solution (viscous aqueous solution) and the pain relief drug (ropivacaine hydrochloride injection; aqueous drug solution), mixed with each other at a volume ratio of 2:1, and means that the final drug concentration is 0.25 wt%.

Summarizing the test results, it was confirmed that the pain of the test group treated with the pain relief drug-containing mixture solution was effectively reduced compared to that of the test group treated with the pain relief drug alone.

Therefore, according to the above-described evaluation results, it can be seen that the pain relief drug-containing mixture solution injected by the surgical kit of the present invention is very effective in providing stable and slow release of the drug in the surgical incision site. In particular, it can be confirmed that the pain relief drug-containing drug mixture solution can be stably prepared by a simple procedure whenever needed, and thus the surgical kit is preferable from an economic point of view.

### [Additional Test Results]

FIG. 6 shows the results of a stability test performed depending on whether the temperature-responsive viscous solution (containing 30 wt% of a temperature-responsive polymer) contained in the pain relief drug- or treatment drug-containing mixture solution was crosslinked. From the test results, it was confirmed that when the content of the temperature-responsive polymer was 25 wt% or less (not shown), the stability of the crosslinked temperature-responsive viscous solution was considerably higher than that of the non-crosslinked temperature-responsive viscous solution, but when the content of the temperature-responsive polymer was 30 wt% or more (see FIG. 6), the stabilities of the crosslinked temperature-responsive viscous solution and the non-crosslinked temperature-responsive viscous solution were all maintained up to 7 days, and thus did not substantially differ from each other.

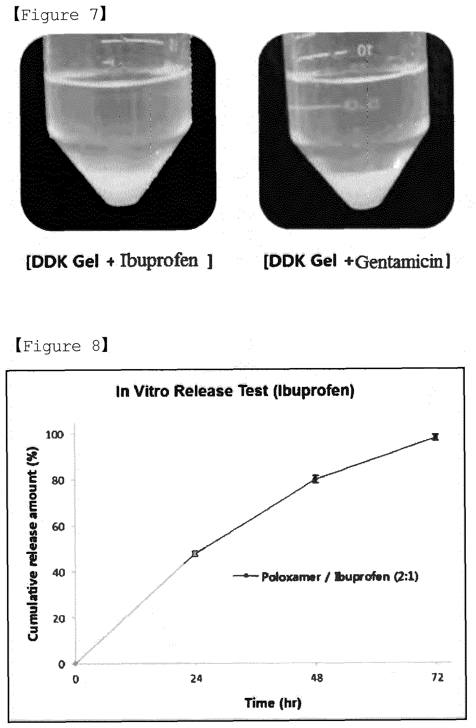

FIG. 7 shows the results for a precipitation test for a pain relief drug-containing mixture solution (left) and a treatment drug-containing mixture solution (right), which contain a crosslinked temperature-responsive viscous solution. In FIG. 7, DDK Gel means a gel composed of poloxamer and crosslinked alginate. From the test results, it was confirmed that in the case of the pain relief drug-containing mixture solution, ibupropene used as the pain relief drug formed a precipitate by reaction with the crosslinking agent CaCl₂, and in the case of the treatment drug-containing mixture solution, the pH of the solution was lowered by gentamicin used as the treatment drug, and thus sodium alginate was precipitated. Namely, when the pain relief drug- or treatment drug-containing mixture solution did not contain the crosslinking CaCl₂ or alginate, no precipitate occurred.

FIG. 8 shows the results of a slow-release test for the pain relief drug (ibuprofen) of the pain relief drug-containing mixture solution. In the slow-release test, an aqueous ibuprofen solution was mixed with a 30 wt% solution of poloxamer, containing no crosslinked alginate, at a volume ratio of 2:1. From the test results, it was confirmed that, in the case of the pain relief drug-containing mixture solution, the drug was released slowly up to 3 days (72 hours).

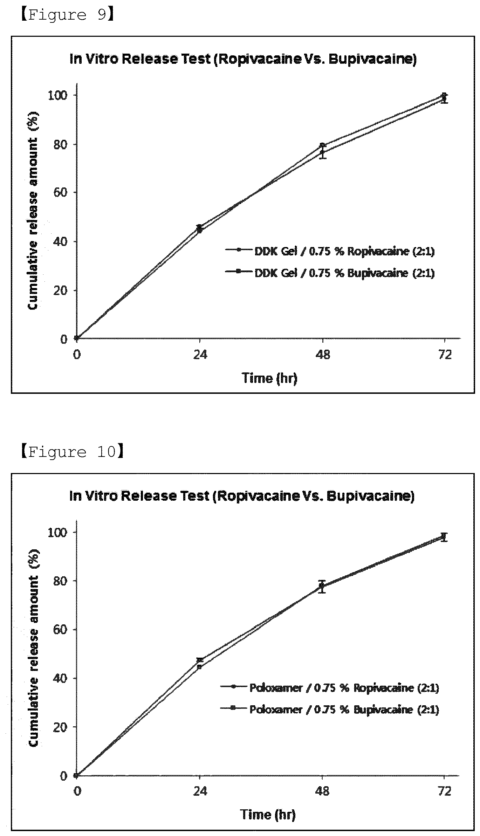

FIGS. 9 and 10 show the results of a slow-release test performed while changing the kinds of drug (ropivacaine vs. bupivacaine) and viscous solution (crosslinked vs. non-crosslinked) in the pain relief drug-containing mixture solution. the slow-release test, a 0.75 wt% aqueous solution of the drug was mixed with the temperature-responsive viscous solution (crosslinked alginate, 30 wt% poloxamer-containing DDK gel vs. 30 wt% poloxamer solution) at a volume ratio of 2:1. From the test results, it was confirmed that the pain relief drug-containing mixture solution was not significantly influenced by the kind of drug or whether or not the temperature-responsive viscous solution was crosslinked, and all the drugs were released slowly up to 3 days (72 hours).

In the present disclosure, the term "crosslinked" means that a crosslinking agent or crosslinked alginate is contained, and the term "non-crosslinked" means that a crosslinking agent or crosslinked alginate is not contained.

### [Description of Reference Numerals]

10: surgical kit; 100: mixture solution injection guide tube; 200: first syringe; 201: first syringe needle; 202: second syringe needle; 300: prefilled syringe; 301: stopper; 302: temperature-responsive viscous solution; 400: syringe connector having an opening/closing means.

## Claims

1. A surgical kit for use in treating or relieving pain at an exposed incision site, comprising:
a prefilled syringe 300 filled with a temperature-responsive viscous solution acting as a stabilization matrix for a pain relief or treatment drug, the prefilled syringe having a structure which is opened and closed by a stopper; and a mixture solution injection guide tube 100 configured to inject a mixture solution containing the pain relief or treatment drug and the temperature-responsive viscous solution in close proximity to the exposed incision site,
wherein the temperature-responsive viscous solution comprises 30 to 40 wt% of a poly(ethylene oxide)/poly(propylene oxide)/poly(ethylene oxide) triblock copolymer containing a poly(ethylene oxide) block and the poly(propylene oxide) block at a ratio of 90:105 to 50:70, and the balance of water for injection, and has a viscosity of 50 to 5,000 cps at 5°C, a viscosity of 1,000,000 to 4,000,000 cps (1 cps corresponds to 1 mPA·s) at 37 °C, and a stickiness of 0.8 N or higher measured using a rotational rheometer at 5°C, wherein the pain relief or treatment drug is used as an aqueous drug solution, and
wherein:
when the pH of the aqueous drug solution is 4-8, the volume ratio between aqueous drug solution and temperature-sensitive viscous solution is 1:0.5 to 5 (aqueous drug solution: temperature-responsive viscous solution), and
when the pH of the aqueous drug solution is 1 to less than 4, the volume ratio between the aqueous drug solution and temperature sensitive viscous solution is 1:4 to 40 (aqueous drug solution: temperature-responsive viscous solution), and
wherein the temperature-responsive viscous solution contains an ionically crosslinked alginate in an amount of 0.05 to 3 wt% of the temperature-responsive viscous solution, and
wherein the drug is a pain relief drug selected from a local anesthetic, an opiate analgesic, a nonsteroidal drug, and
wherein the aqueous drug solution and the temperature-responsive viscous solution are combined to give a final drug mixture with a concentration in the range of 0.1 to 0.5 wt%; and wherein viscosity is measured with a Brookfield viscometer under conditions of #4 spindle at 5°C and #7 spindle at 37°C in accordance with method 2 (rotational viscometer method) described in the Korean Pharmacopoeia.

2. A surgical kit as claimed in claim 1, wherein the surgical kit further comprises a syringe needle 201 for a first syringe 200, and wherein the first syringe 200 is preassembled with a syringe needle 201 or connected with the syringe needle 201 immediately before use, and the syringe needle 201 is separated from the first syringe 200 after being filled with the pain relief or treatment drug aqueous solution.

3. A surgical kit as claimed in claim 1, wherein the temperature-responsive viscous solution comprises 30 to 40 wt% of a poly(ethylene oxide)/poly(propylene oxide)/poly(ethylene oxide) triblock copolymer , 0.005 to 0.1 wt% of a crosslinker, 0.05 to 3 wt% of alginic acid or alginate and the remainder of water.

4. A surgical kit as claimed in claim 1, further comprising a syringe connector 400 having an inner diameter of 10 mm or less.

5. A surgical kit as claimed in claim 1, further comprising an absorption means for absorbing and removing water remaining around the incision site.

## Patentansprüche

1. Chirurgie-Kit zur Verwendung beim Behandeln oder Lindern von Schmerzen an einer freiliegenden Inzisionsstelle, umfassend:
eine vorgefüllte Spritze 300, die mit einer temperaturreaktiven viskosen Lösung gefüllt ist, die als Stabilisierungsmatrix für ein Arzneimittel zur Linderung oder Behandlung von Schmerzen dient, wobei die vorgefüllte Spritze eine Struktur aufweist, die durch einen Stopfen geöffnet und geschlossen wird; und ein Führungsrohr 100 zum Injizieren einer Mischlösung, das konfiguriert ist, um eine Mischlösung, die das Arzneimittel zur Linderung oder Behandlung von Schmerzen und die temperaturreaktive viskose Lösung enthält, in unmittelbarer Nähe der freiliegenden Inzisionsstelle zu injizieren,
wobei die temperaturreaktive viskose Lösung 30 bis 40 Gew.-% eines Poly(ethylenoxid)/Poly(propylenoxid)/Poly(ethylenoxid)-Triblockcopolymers umfasst, das einen Poly(ethylenoxid)-Block und den Poly(propylenoxid)-Block in einem Verhältnis von 90:105 bis 50:70, und als Rest Wasser zur Injektion enthält und eine Viskosität von 50 bis 5.000 cps bei 5 °C, eine Viskosität von 1.000.000 bis 4.000.000 cps (1 cps entspricht 1 mPA·s) bei 37 °C und eine Klebrigkeit von 0,8 N oder höher, gemessen unter Verwendung eines Rotationsrheometers bei 5 °C, aufweist, wobei das Arzneimittel zur Linderung oder Behandlung von Schmerzen als wässrige Arzneimittellösung verwendet wird, und
wobei:
wenn der pH-Wert der wässrigen Arzneimittellösung 4-8 beträgt, das Volumenverhältnis zwischen wässriger Arzneimittellösung und temperaturempfindlicher viskoser Lösung 1:0,5 bis 5 (wässrige Arzneimittellösung: temperaturreaktive viskose Lösung) beträgt, und
wenn der pH-Wert der wässrigen Arzneimittellösung 1 bis weniger als 4 beträgt, das Volumenverhältnis zwischen der wässrigen Arzneimittellösung und der temperaturempfindlichen viskosen Lösung 1:4 bis 40 (wässrige Arzneimittellösung: temperaturreaktive viskose Lösung) beträgt, und
wobei die temperaturreaktive viskose Lösung ein ionisch vernetztes Alginat in einer Menge von 0,05 bis 3 Gew.-% der temperaturreaktiven viskosen Lösung enthält, und
wobei das Arzneimittel ein Schmerzlinderungsmittel ist, ausgewählt aus einem Lokalanästhetikum, einem Opiatanalgetikum, einem nichtsteroidalen Arzneimittel und
wobei die wässrige Arzneimittellösung und die temperaturreaktive viskose Lösung kombiniert werden, um eine endgültige Arzneimittelmischung mit einer Konzentration im Bereich von 0,1 bis 0,5 Gew.-% zu ergeben; und
wobei Viskosität mit einem Brookfield-Viskosimeter unter Bedingungen von #4 Spindel bei 5°C und #7 Spindel bei 37 °C gemäß Verfahren 2 (Rotationsviskosimeter-Verfahren) gemessen wird, die im Koreanischen Arzneibuch beschrieben ist.

2. Chirurgie-Kit nach Anspruch 1, wobei das Chirurgie-Kit weiter eine Spritzennadel 201 für eine erste Spritze 200 umfasst, und wobei die erste Spritze 200 mit einer Spritzennadel 201 vormontiert ist oder unmittelbar vor Verwendung mit der Spritzennadel 201 verbunden wird und die Spritzennadel 201 von der ersten Spritze 200 getrennt wird, nachdem sie mit der wässrigen Lösung des Arzneimittels zur Linderung oder Behandlung von Schmerzen gefüllt worden ist.

3. Chirurgie-Kit nach Anspruch 1, wobei die temperaturreaktive viskose Lösung 30 bis 40 Gew.-% eines Poly(ethylenoxid)/Poly(propylenoxid)/Poly(ethylenoxid)-Triblockcopolymers, 0,005 bis 0,1 Gew.-% eines Vernetzungsmittels, 0,05 bis 3 Gew.-% Alginsäure oder Alginat und als Rest Wasser umfasst.

4. Chirurgie-Kit nach Anspruch 1, das weiter einen Spritzenverbinder 400 umfasst, der einen Innendurchmesser von 10 mm oder weniger aufweist.

5. Chirurgie-Kit nach Anspruch 1, das weiter ein Absorptionsmittel zum Absorbieren und Entfernen von um die Inzisionsstelle herum verbleibendem Wasser umfasst.

## Revendications

1. Kit chirurgical destiné à être utilisé pour traiter ou soulager la douleur au niveau d'un site d'incision exposé, comprenant :
une seringue préremplie 300 remplie d'une solution visqueuse sensible à la température agissant comme matrice de stabilisation pour un médicament antidouleur ou de traitement, la seringue préremplie présentant une structure qui est ouverte et fermée par un bouchon ; et un tube de guidage d'injection de solution de mélange 100 configuré pour injecter une solution de mélange contenant le médicament antidouleur ou de traitement et la solution visqueuse sensible à la température à proximité immédiate du site d'incision exposé,
dans lequel la solution visqueuse sensible à la température comprend 30 à 40 % en poids d'un copolymère oxyde de polyéthylène/oxyde de polypropylène/oxyde de polyéthylène triséquencé contenant une séquence oxyde de polyéthylène et la séquence oxyde de polypropylène selon un rapport de 90:105 à 50:70, et le reste étant de l'eau pour injection, et présente une viscosité de 50 à 5 000 cps à 5 °C, une viscosité de 1 000 000 à 4 000 000 cps (1 cps correspond à 1 mPA·s) à 37 °C, et une adhésivité supérieure ou égale à 0,8 N mesurée à l'aide d'un rhéomètre rotatif à 5 °C, dans lequel le médicament anti-douleur ou de traitement étant utilisé sous forme de solution médicamenteuse aqueuse, et
dans lequel :
lorsque le pH de la solution médicamenteuse aqueuse est de 4-8, le rapport volumique entre la solution médicamenteuse aqueuse et la solution visqueuse sensible à la température est 1:0,5 à 5 (solution médicamenteuse aqueuse : solution visqueuse sensible à la température), et
lorsque le pH de la solution médicamenteuse aqueuse est de 1 à moins de 4, le rapport volumique entre la solution médicamenteuse aqueuse et la solution visqueuse sensible à la température est 1:4 à 40 (solution médicamenteuse aqueuse : solution visqueuse sensible à la température), et
dans lequel la solution visqueuse sensible à la température contient un alginate réticulé ioniquement en une quantité de 0,05 à 3 % en poids de la solution visqueuse sensible à la température, et
dans lequel le médicament est un médicament anti-douleur choisi parmi un anesthésique local, un anti-douleur opiacé, un médicament non stéroïdien et
dans lequel la solution médicamenteuse aqueuse et la solution visqueuse sensible à la température sont combinées pour donner un mélange final de médicament avec une concentration comprise dans la plage de 0,1 à 0,5 % en poids ; et
dans lequel la viscosité est mesurée avec un viscosimètre Brookfield dans des conditions de tige n°4 à 5 °C et de tige n°7 à 37 °C conformément au procédé 2 (procédé de viscosimètre rotatif) décrite dans la Pharmacopée coréenne.

2. Kit chirurgical selon la revendication 1, dans lequel le kit chirurgical comprend en outre une aiguille de seringue 201 pour une première seringue 200, et dans lequel la première seringue 200 est préassemblée avec une aiguille de seringue 201 ou connectée à l'aiguille de seringue 201 immédiatement avant utilisation, et l'aiguille de seringue 201 est séparée de la première seringue 200 après avoir été remplie avec la solution médicamenteuse aqueuse anti-douleur ou de traitement.

3. Kit chirurgical selon la revendication 1, dans lequel la solution visqueuse sensible à la température comprend 30 à 40% en poids d'un copolymère oxyde de polyéthylène/oxyde de polypropylène/oxyde de polyéthylène triséquencé, 0,005 à 0,1 % en poids d'un agent de réticulation, 0,05 à 3 % en poids d'acide alginique ou d'alginate et le reste étant de l'eau.

4. Kit chirurgical selon la revendication 1, comprenant en outre un connecteur de seringue 400 présentant un diamètre intérieur inférieur ou égal à 10 mm.

5. Kit chirurgical selon la revendication 1, comprenant en outre un moyen d'absorption pour absorber et éliminer l'eau restant autour du site d'incision.
